# EUROPEAN PATENT APPLICATION

(11) **EP 1 417 987 A1**
(43) Date of publication of application: **12.05.2004**
(21) Application number: 02717161.0
(22) Date of filing: 15.04.2002
(51) Int. Cl.: A61N 5/06

(54) **OPTICAL MEDICAL TREATMENT DEVICE USING POLARIZATION**

(30) Priority: 26.04.2001 JP 2001128745
(71) Applicant: Yamada, Takashi, Fukuoka-shi, Fukuoka 811-1122 (JP)
(72) Inventor: Yamada, Takashi, Fukuoka-shi, Fukuoka 811-1122 (JP)
(74) Representative: Thinat, Michel
(86) International application number: PCT/JP2002/003752
(87) International publication number: WO 2002/087699

(57) **Abstract**

Polarized light-based phototherapy equipment according to the present invention is designed to focus light from a halogen lamp (1) using a light-focusing lens (6), and then to direct the focused light to a revolving plate (3) that is formed with a pair of cutouts. The revolving plate (3) is rotated at 2, 500 rpm. Accordingly, the focused light is significantly traveled through the revolving plate (3) at the cutouts, but is predominantly blocked from passing through the revolving plate (3) in a range other than the cutouts. As a result, the light is emitted to an optical fiber (7) in a pulse-like manner at a period of 0.012 seconds. A polarizing filter (8) at an end of the optical fiber (7) polarizes the pulse-like light. The polarized light is irradiated onto an affected area.

## Description

### Technical Field

This invention relates to equipment operable to largely polarize visible light or infrared light, and to irradiate the polarized light to an affected area and the skin, thereby curing muscular rheumatism and spasm.

### Art of the Background

There has heretofore been widely known phototherapy equipment designed to polarize light from low output laser such as a He-Ne laser and a diode laser, and to irradiate the polarized laser wave to an affected area. It is well known that optical irradiation has beneficial effects to remove and inhibit pain. However, the optical irradiation is low in output, and is insufficient to exercise perfect effects from practical viewpoints. A laser oscillator is constructed to polarize the output of a high-output laser such as an Nd-YAG laser before introducing the polarized output to the affected area through a fiber. In the fiber, the laser output is reflected against the fiber. As a result, the laser light irradiated to the affected area is no longer polarized light from a macroscopic viewpoint, and is less effective in cure.

In order to smooth out the above two different shortcomings, an improved method has more operatively been carried out. According to such a method, a halogen lamp is used as a light source to ensure sufficient output. In addition, a polarizing filter usually processes light at a position immediately before the light is irradiated onto the affected area. However, the above method brings about disturbances due to an increase in temperature at the affected area where the light is irradiated. Therefore, the light must be reduced in output. More specifically, when a halogen lamp having output of 1.8 W continuously irradiates for two seconds, then the irradiated area (affected area) increases in temperature. Accordingly, the light irradiation must be stopped.

It has heretofore been thought that polarized light-based therapy provides effects related to thermal effects. However, it has been proved that lower output laser light (10 to 20 mW) without an increase in temperature is effective in carrageenan-caused artificial edema of a mouse. In addition, it has been reported that the same laser light is effective in restraining an action potential of cat's nerve cells. Moreover, it has been acknowledged that the polarized output of a light-emitting diode is effective in accelerating injury cure. The irradiated area was measured using a thermo-camera to check for a change in temperature of the irradiated area. The measurement showed no increase in temperature. Accordingly, the present inventor is convinced that the effectiveness of the polarized light-based therapy involves no increase in temperature.

### Disclosure of the Invention

In view of the above, the present invention has been devised. An object of the present invention is to provide polarized light-based phototherapy equipment operable to irradiate polarized light with a time-based stress, thereby allowing sufficient electric field intensity effective in medical treatment to be imparted to an affected area without the occurrence of heat-caused disturbances to the affected area.

In order to achieve the above object, the present invention provides the following:
1) polarized light-based phototherapy equipment designed to polarize light from a light source using a polarizing filter, and to irradiate the polarized light onto an affected area, comprising: a light-strengthening/weakening means operable to permit the luminous intensity of irradiated and polarized light to alternate between strong and weak on a cyclic basis, whereby effective, polarized light having increased intensity can be irradiated onto the affected area without a burn injury to the affected area.
2) polarized light-based phototherapy equipment as defined in the above paragraph 1), wherein the light-strengthening/weakening means is operable to emit the polarized light intermittently.
3) polarized light-based phototherapy equipment as defined in the above paragraph 1), wherein the light-strengthening/weakening means is operable to intermittently open and close an optical path using a shutter plate before.or after polarization in order to permit the light to alternate between strong and weak on a cyclic basis.
4) polarized light-based phototherapy equipment as defined in the above paragraph 2), wherein the light-strengthening/weakening means is operable to intermittently open and close an optical path using a shutter plate before or after polarization in order to permit the light to alternate between strong and weak on a cyclic basis.
5) polarized light-based phototherapy equipment as defined in the above paragraph 1), wherein the light-strengthening/weakening means is operable either to permit light emission from the light source to alternate between strong and weak on a cyclic basis or to cyclically stop the light emission, whereby the irradiated and polarized light has the intensity alternating between strong and weak on a cyclic basis.
6) polarized light-based phototherapy equipment as defined in the above paragraph 1), wherein the light-strengthening/weakening means comprises a mirror reflector operable to reflect the light from the light source, a fluctuating means operable to impart either vibration or rotation to the mirror reflector in order to permit the light reflected against the mirror reflector to be cyclically reflected in multiple directions, and a light-restricting means operable to permit only light falling within a range of a limited angle among the reflected light to be emitted to a polarizing filter.
7) polarized light-based phototherapy equipment as defined in any one of the above paragraphs 1) to 6), wherein an optical fiber emits pre-polarized light to the polarizing filter.

### Brief Description of the Drawings

FIG. 1 is a descriptive illustration showing a first embodiment;
FIG. 2 is an elevation view illustrating a revolving plate according to the first embodiment;
FIG. 3 is a descriptive illustration showing a second embodiment; and
FIG. 4 is a descriptive illustration showing a third embodiment.

### Best Mode for Embodying the Invention

A halogen lamp, a light-emitting diode, and an incandescent lamp may be used as a light source according to the present invention. Light from the light source may use a waveband of visible light or infrared rays.

An optical path according to the present invention for emitting light to a polarizing filter (polarizing plate) includes an optical fiber and a mirror surface, whereby the light travels through the air. The optical fiber is more useful as a method for emitting the light to a predetermined position, a narrow spot, and a complicated location.

The polarizing filter according to the present invention is preferably positioned adjacent to a location where polarized light is irradiated onto an affected area.

Strong and weak-alternating variations in luminous intensity according to the present invention include two different cases. One of the cases provides the luminous intensity in a pulse-like manner when the luminous intensity decreases to nearly zero. The other case pulsates the light intensity when lower luminous intensity decreases.

A method (mechanism) according to the present invention for irradiating the polarized light that has the luminous intensity alternating between strong and weak includes two different methods. One of the methods is to intermittently block or accentually control either pre-polarized or post-polarized light of emitted light in a mechanical manner. Another method is to either intermittently (in a pulse-like manner) emit the light from the light source or electrically control the light emission in such a manner that the intensity of the emitted light alternates between strong and weak. In the intermittent light emission according to the present invention, a practical ratio of stronger emission (open) time to weaker (stop, close) time is approximately 3: 7. Pursuant to the present invention, the light emission having a 0.2 seconds (5 Hz or greater) or smaller open-close period or strong and weak-alternating period brings about no burn injures, and imparts a high-electric field to the affected area. As a result, the light emission having such a period is practical and effective in medical treatment.

Embodiments of the present invention are now described with reference to the accompanying drawings.

### First embodiment

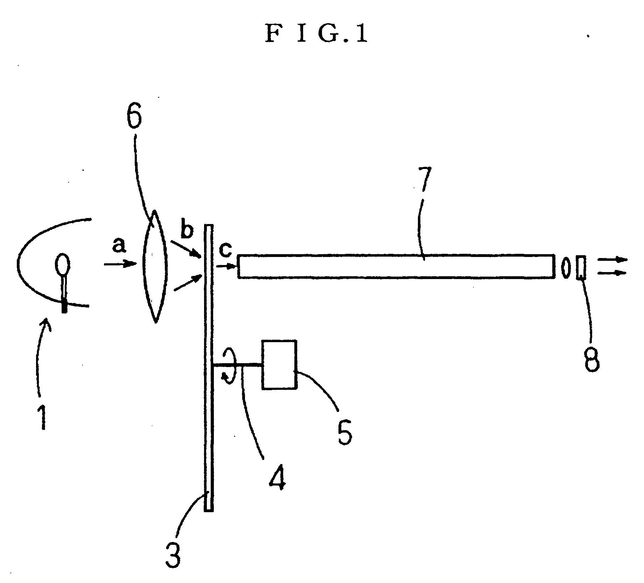

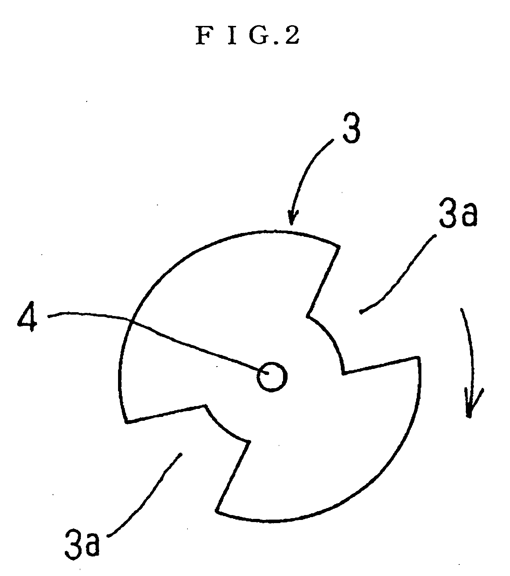

FIGS. 1 and 2 illustrate a first embodiment that provides a test model according to the present invention. The test model uses a 300-W halogen lamp as a light source. A revolving plate for use in intermittent blockage is rotated to emit light from the halogen lamp in an intermittent (pulse-like) manner. The emitted light enters an optical fiber. A polarizing filter is disposed on the optical fiber at a rear end thereof.

FIG. 1 is a descriptive illustration showing the present embodiment.

FIG. 2 is an elevation view illustrating the revolving plate according to the present embodiment.

In FIGS. 1 and 2, reference numerals 1, 3, 3a, 4, 5, 6, 7, and 8 denote the halogen lamp having the output of 300 W, the round-shaped, revolving plate for use in intermittent blockage, a cutout of the revolving plate, a revolving shaft of the revolving plate, a motor operable to rotate the revolving plate at 2,500 rpm, a light-focusing lens, the optical fiber, and the polarizing filter (polarizing plate), respectively.

Pursuant to the present embodiment, light directed from the halogen lamp 1 to the light-focusing lens 6, as illustrated by arrow "a", is focused as illustrated by arrow "b" by the light-focusing lens 6. The focused light is directed to the revolving plate 3 that is defined with a pair of cutouts 3a as illustrated in FIG. 2. The revolving plate 3 is rotated at 2, 500 rpm. Accordingly, the focused light is predominantly blocked from passing through the revolving plate 3, while being traveled through the revolving plate 3 at the cutouts 3a and at only part of an outer circumference of the revolving plate 3 except for the cutouts 3a. As a result, the focused light alternates between strong and weak, which is closer to switching (pulse). Such strong and weak-alternating light is directed into the optical fiber 7, as illustrated by arrow C, and is then brought to a position near an affected area through the optical fiber 7. The strong and weak-alternating light is polarized when passing through the polarizing filter 8. The polarized light is irradiated onto the affected area. The polarized light has a strong and weak-alternating period of 12 milliseconds.

The polarized intermittent (pulse-like) light having the period of 0.012 seconds according to the present embodiment was irradiated onto the affected area for two minutes, but no feeling of heat was produced. Instead, a comfortable stimulus like acupuncture and moxibustion was continuously produced immediately after the irradiation, and the effectiveness of pain removal was highly acknowledged. The stimulus was momentarily felt when the irradiation was stopped because of a feeling of heat upon the continuous (not intermittent) irradiation for some two seconds. It is empirically known that the absence of such a stimulus results in low effectiveness.

As described above, the irradiation of the polarized light according to the present embodiment was useful in medical treatment. In addition, the same irradiation brought about few burn injuries at the affected area.

### Second embodiment

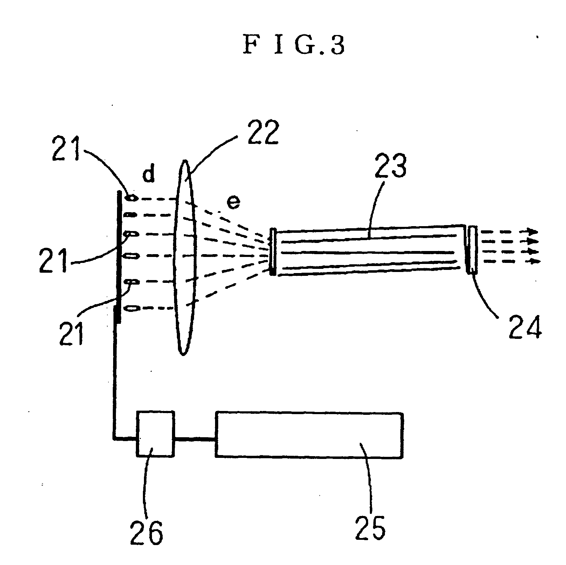

A second embodiment as illustrated in FIG. 3 employs fifty to one hundred infrared-ray-emitting diodes as light sources, and further employs a switching circuit to provide intermittent light emission. The switching circuit is operable to permit the voltage applied to the infrared-ray-emitting diodes to be switched at 100 Hz using a transistor.

FIG. 3 is a descriptive illustration showing the present embodiment.

In FIG. 3, reference numerals 21, 22, 23, 24, 25, and 26 denote the infrared-ray-emitting diode of some 40 mW, a light-focusing lens, an optical fiber, a polarizing filter, a DC power supply for the diodes 21 and a switching circuit 26, and the switching circuit employing the transistor to switch power supply passages of the diodes 21 at 100 Hz.

Pursuant to the present embodiment, the fifty to one hundred infrared-ray-emitting diodes, each nearly 40 mW, are employed as light sources. As a result, infrared rays of maximum 2 to 4 W can be emitted. The switching circuit 26 electrically limits (deactivation, a non-voltage-applied state) the number of the infrared-ray-emitting diodes 21 to be used. As a result the infrared rays having the output smaller than the maximum value can be emitted.

The switching circuit 26 was operated to permit the infrared-ray-emitting diodes 21 to emit the light in a pulse-like manner at periods of 3-millisecond light emission and 7-milisecond stop. As illustrated by dashed lines "d", the light is directed to the light-focusing lens 22. The light-focusing lens 22 condenses the light. As illustrated by dashed line "e", the condensed light enters the optical fiber 23. The entering light is ultimately irradiated onto an affected area through the polarizing filter 24 at an end of the optical fiber 23.

Similarly to the previous embodiment, the irradiation continued to produce a feeling of acupuncture and moxibustion-like stimulus, and the effectiveness of pain removal was acknowledged. No feeling of excessive heat was produced at the affected area.

### Third embodiment

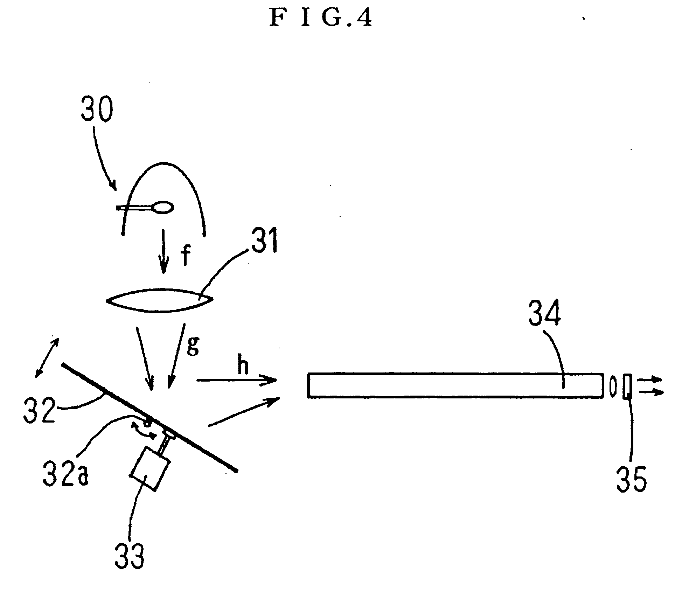

A light-strengthening/weakening means according to a third embodiment as illustrated in FIG. 4 includes a mirror reflector 32, a vibrator 33 operable to vibrate the mirror reflector to vary reflecting directions, a revolving axis 32a of the mirror reflector 32, and a control means operable to lead only part of reflected light into an optical fiber 34. The light emitted through the optical fiber 34 is polarized at a rear end of the optical fiber 34 using a polarizing plate 35. The polarized light is irradiated onto an affected area.

FIG. 4 is a descriptive illustration showing the present embodiment.

In FIG. 4, reference numerals 30, 31, 32, 32a, 33, 34, and 35 denote a 300-W halogen lamp, a light-focusing lens, the mirror reflector, the revolving axis of the mirror reflector, the vibrator operable to vibrate the mirror reflector 32 cyclically about the axis 32a, the optical fiber, and the polarizing filter.

As illustrated by arrow "f" of FIG. 4, pursuant to the present embodiment, light from the halogen lam 30 is directed to the light-focusing lens 31, at which the light is focused. As illustrated by arrow "g", the focused light is directed to the mirror reflector 32, against which the light is reflected. When the vibrator 33 vibrates the direction of the reflected light at a 50-Hz vibratory frequency, then the mirror reflector 32 is vibrated about the axis 32a at the same 50-Hz vibratory frequency. The reflected light is varied within a range of such an angle. As illustrated by arrow "h", only part of the reflected light, which falls within a predetermined range, enters the optical fiber 34. The light is emitted through the optical fiber 34 at a period of 0.02 seconds, and is then polarized by the polarizing filter 35. The polarized light is irradiated in a pulse-like manner.

The irradiation of the polarized light according to the present embodiment resulted in no burn injuries at the affected area. Similarly to the previous embodiments, the same irradiation was acknowledged being effective in medical treatment.

### Possible Utilization in Industry

As described above, the polarized light-based phototherapy equipment according to the present invention is operable to irradiate polarized light with a cyclic stress. As a result, high-intensity light useful in medical treatment can be irradiated for a long time with little excess heat at an affected area. In addition, the polarized light-based phototherapy equipment according to the present invention provides increased irradiation in an intermittent manner, thereby providing an acupuncture and moxibustion-like stimulus. As a result, the irradiation is highly effective in pain removal.

## Claims

1. Polarized light-based phototherapy equipment designed to polarize light from a light source using a polarizing filter, and to irradiate the polarized light onto an affected area, comprising a light-strengthening/weakening means operable to permit luminous intensity of irradiated and polarized light to alternate between strong and weak on a cyclic basis, whereby effective, polarized light having increased intensity can be irradiated onto the affected area without a burn injury to the affected area.

2. Polarized light-based phototherapy equipment as defined in claim 1, wherein said light-strengthening/weakening means is operable to emit the polarized light intermittently.

3. Polarized light-based phototherapy equipment as defined in claim 1, wherein said tight-strengthening/weakening means is operable to intermittently open and close an optical path using a shutter plate before or after polarization in order to permit the light to alternate between strong and weak on a cyclic basis.

4. Polarized light-based phototherapy equipment as defined in claim 2, wherein said light-strengthening/weakening means is operable to intermittently open and close an optical path using a shutter plate before or after polarization in order to permit the light to alternate between strong and weak on a cyclic basis.

5. Polarized light-based phototherapy equipment as defined in claim 1, wherein said light-strengthening/weakening means is operable either to permit light emission from the light source to alternate between strong and weak on a cyclic basis or to cyclically stop the light emission, whereby the irradiated and polarized light has the intensity alternating between strong and weak on a cyclic basis.

6. Polarized light-based phototherapy equipment as defined in claim 1, wherein said light-strengthening/weakening means comprises a mirror reflector operable to reflect the light from the light source, a fluctuating means operable to impart one of vibration and rotation to the mirror reflector in order to permit the light reflected against the mirror reflector to be cyclically reflected in multiple directions, and a light-restricting means operable to permit only light falling within a range of a limited angle among the reflected light to be emitted to said polarizing filter.

7. Polarized light-based phototherapy equipment as defined in any one of claims 1 to 6, wherein an optical fiber emits pre-polarized light to said polarizing filter.
